# EUROPEAN PATENT SPECIFICATION

(11) **EP 3 331 429 B1**
(45) Date of publication and mention of the grant of the patent: **02.09.2020**
(21) Application number: 16821869.1
(22) Date of filing: 05.07.2016
(51) Int. Cl.: A61B 5/00, A61B 5/103, A61B 5/11, A61B 8/00, A61B 8/08

(54) **SENSOR ASSEMBLY FOR USE WITH A POSITIONAL TRACKING SYSTEM AND METHOD OF MANUFACTURE**
SENSORANORDNUNG ZUR VERWENDUNG MIT EINEM POSITIONSVERFOLGUNGSSYSTEM UND VERFAHREN ZUR HERSTELLUNG
ENSEMBLE DE DÉTECTION DESTINÉ À ÊTRE UTILISÉ AVEC UN SYSTÈME DE SUIVI DE POSITION, ET PROCÉDÉ DE FABRICATION

(30) Priority: 06.07.2015 US 201562231369 P
(43) Date of publication of application: 13.06.2018
(73) Proprietor: METRITRACK, INC., Hillside, IL 60162 (US)
(72) Inventor: CALUSER, Calin, Glen Ellyn, IL 60137 (US)
(74) Representative: Klang, Alexander H.
(86) International application number: PCT/US2016/040950
(87) International publication number: WO 2017/007752

(56) References cited:
- WO-A2-2014/145814
- US-A1- 2009 235 396
- US-A1- 2012 022 376
- US-A1- 2012 165 645
- US-A1- 2013 079 670
- US-A1- 2013 158 404
- US-A1- 2015 011 858
- US-B1- 8 083 693

## Description

### CROSS REFERENCE TO RELATED APPLICATION

### BACKGROUND OF THE INVENTION

Embodiments of the invention relate generally to medical imaging and, more particularly, to a three-dimensional (3D) positional registration system incorporating one or more sensor assemblies that are positionable at anatomical reference points on or within the patient body and used to track the position of the respective anatomical reference points during repeat examinations.

Ultrasound imaging systems transmit sound waves of very high frequency (e.g., 1 MHz to 20 MHz) into the patient's body and the echoes scattered from structures in the patient's body are processed to create and display images and information related to these structures. Ultrasound is an important imaging modality for medical diagnostic purposes and as a guidance tool for diagnostic or screening purposes and for therapeutic procedures, such as, for example soft tissue needle biopsy, tumor ablation, and the like. A diagnostic ultrasound examination is performed to address a specific medical concern and provide additional evaluation to reach the diagnosis. For example, in breast ultrasound, a diagnostic examination can be performed to evaluate a palpable lump or focal pain or evaluate a lesion detected with other modality like mammography or MRI. 2D free hand ultrasound imaging, the most common technique used today, represents a slice through the region of interest. During a breast ultrasound procedure, for example, the radiologist, technician, or other medical professional (the "operator") places an ultrasound transducer over a region of interest of the breast and is then able to view a real-time ultrasound image that is output on a display.

Ultrasound procedures are highly dependent on the device user's experience and training. The vast majority of ultrasound examinations are conducted free hand, with the operator holding the ultrasound transducer in one hand and use the other hand to operate the ultrasound machine controls. The operator pauses movement of the ultrasound probe upon viewing a possible lesion, tumor, or other specious finding in a displayed image and will then manually mark the location of the suspicious finding in the image, often by entering alpha numerical characters or graphical signs.

Position recording of suspicious findings is important, especially for small targets and/or multiple targets identified in an image or series of acquired images. The smaller the tumor is before treatment, the higher the probability of long term patient survival or cure. However, small tumors are difficult to find in a patient's body and difficult to differentiate from other structures or artifacts in the same region. Many times a suspicious small finding can coexist in the same region with multiple benign findings (cysts, solid benign nodules, etc.) with similar appearance, which may create confusion during a follow-up examination and may lead to missing the suspicious lesion. As imaging diagnostic devices provide ever greater detail and sub-millimeter resolution, accurate position registration and mapping of lesions is becoming increasingly important in order to take advantage of the increased capabilities.

Because of the importance of properly locating targets in an acquired ultrasound image, it is desirable to obtain the instant recording of target coordinates seen in the ultrasound image in relation to the anatomical reference (for example, a nipple) and the simultaneous recording of the ultrasound probe position. Although ultrasound guidance systems and devices do exist, known systems do not offer a practical and accurate solution to mapping targets in 2D or 3D images with real time correction for movement of the patient's body between images and motion of deformable tissue between images.

As a result, it would be desirable to have a positional tracking system that incorporates one or more sensor assemblies that facilitate positional tracking of common anatomical reference points on the patient during repeat examinations. It would further be desirable for such a system to improve the accuracy in tracking the location of targets or lesions within deformable structures and account for patient body movement between image frames.

WO 2014/145814 A2 discloses a sensor attachment for use with three dimensional ultrasound mapping devices. It discloses that one or more sensors may be attached to specific locations on the body, such as the nipple and sternum, to enabling accurate recording of target information, including location and size.

US 2012/165645 A1 discloses a system, method, and portable device for monitoring physiological parameters of a person in the field. It discloses connecting a monitoring device of a biosensor system to a garment where one or more sensors are attached or integrated into the garment.

### BRIEF DESCRIPTION OF THE INVENTION

Embodiments of the invention are directed to a three-dimensional (3D) positional registration system incorporating one or more sensor assemblies that are positionable at anatomical reference points on or within the patient body and used to track the position of the respective anatomical reference points during repeat examinations.

In accordance with the invention, an assembly for facilitating placement of a positional sensor as set forth in claim 1, a method of manufacturing an assembly for facilitating placement of a positional sensor as set forth in claim 7 and a three-dimensional position tracking system as set forth in claim 12 are provided. The assembly for facilitating placement of a positional sensor inter alia includes a mounting base comprising at least one flexible tab, a mounting flange forming a bottom surface of the mounting base, and a ledge positioned between a top surface of the at least one flexible tab and the mounting flange. A sensor housing is positioned on the ledge and rotatably engaged with the mounting base. The sensor housing has a cavity sized to receive a positional sensor and the at least one flexible tab engages an upward-facing surface of the sensor housing.

The method of manufacturing an assembly for facilitating placement of a positional sensor inter alia includes forming a mounting base having at least one flexible tab, a mounting flange, and a ledge positioned between the mounting flange and a top surface of the mounting base. The method also includes forming a sensor housing having a first portion sized to seat on the ledge of the mounting base and a second portion having a cavity formed therein to receive a positional sensor, the sensor housing rotatably engageable with the mounting base. The at least one flexible tab is sized to engage an upward-facing surface of the first portion of the sensor housing.

The 3D position tracking system inter alia includes a positional tracking system and a first sensor assembly having a mounting base comprising a bottom flange, at least one flexible tab, and a ledge positioned therebetween. A sensor housing is positioned on the ledge and rotatably engaged with the mounting base. A sensor is positioned within a cavity formed in the sensor housing and a sensor wires extending outward from an opening formed in the sensor housing, the sensor wire having a first end coupled to the magnetic sensor and a second end coupled to the positional tracking system. The positional tracking system is configured to track a real-time position of the sensor.

Various other features and advantages will be made apparent from the following detailed description and the drawings.

### BRIEF DESCRIPTION OF THE DRAWINGS

The drawings illustrate preferred embodiments presently contemplated for carrying out the invention.

In the drawings:
FIG. 1 depicts an overview illustration of an imaging system that includes an ultrasound device and a 3D mapping display system (TDMD), according to an embodiment of the invention.
FIG. 2 is a schematic diagram illustrating the relative positioning of sensor assemblies of the TDMD of FIG. 1 during an exemplary breast ultrasound examination.
FIG. 3 illustrates an exemplary body diagram and ultrasound image frame displayed on the display of the imaging system of FIG. 1.
FIG. 4 is a side perspective view of a magnetic sensor assembly according to an embodiment of the invention.
FIG. 5 is an exploded perspective view of the magnetic sensor assembly of FIG. 4.
FIG. 6 is a bottom perspective view of the magnetic sensor assembly of FIG. 4.
FIG. 7 is a bottom view of the magnetic sensor assembly of FIG. 4.
FIG. 8A is a cross-sectional view of the magnetic sensor assembly of FIG. 4.
FIG. 8B is a detail view of a portion of FIG. 8A.
FIG. 9 is a top view of the mounting base of the magnetic sensor assembly of FIG. 4.
FIG. 10 is a side view of the mounting base of the magnetic sensor assembly of FIG. 4.
FIG. 11 is a top view of the disk portion of the sensor housing, according to an embodiment of the invention.
FIG. 12 is a bottom view of the disk portion of the sensor housing, according to an embodiment of the invention.
FIG. 13 is a side view of the protrusion portion of the sensor housing, according to an embodiment of the invention.
FIG. 14 is a top view of the protrusion portion of the sensor housing, according to an embodiment of the invention.
FIG. 15 is a bottom view of the protrusion portion of the sensor housing, according to an embodiment of the invention.
FIG. 16 is a side perspective view of a magnetic sensor assembly, according to another embodiment of the invention.
FIG. 17 is a side perspective view of a magnetic sensor assembly, according to another embodiment of the invention.
FIG. 18 is an exploded perspective view of the magnetic sensor assembly of FIG. 17.
FIG. 19 is a schematic representation of the relative positioning of the magnetic sensor assembly of FIG. 17 on a patient with the use of an optional alignment member.
FIG. 20 is a front view of the magnetic sensor assembly of FIG. 17.
FIG. 21 is a cross-sectional view of FIG. 20.
FIG. 22 is a bottom view of the mounting base of the magnetic sensor assembly of FIG. 17.
FIG. 23 is a rear perspective view of the sensor housing of the magnetic sensor assembly of FIG. 17.
FIG. 24 is a side perspective view of an optical sensor assembly according to an embodiment of the invention.

### DETAILED DESCRIPTION

The operating environment of the various embodiments of the invention are described below with respect to a 2D ultrasound imaging system. However, it will be appreciated by those skilled in the art that the invention the concepts disclosed herein may be extended to 3D ultrasound imaging systems as well as images obtained with a different imaging modality or combination of imaging modalities, such as, for example, x-ray, CT or MRI. Images separately acquired using any of these modalities may be co-registered in space with positional registration to the same anatomical sensor(s) or marker(s) and displayed in a similar manner as described below for ultrasound images. Further, embodiments of the invention may be used for ultrasound breast cancer screening or diagnostic breast ultrasound exams. Additionally, the techniques disclosed herein may be extended to image data acquired from other regions in the body such as, for example, the eye, liver, abdomen, neck, and kidneys.

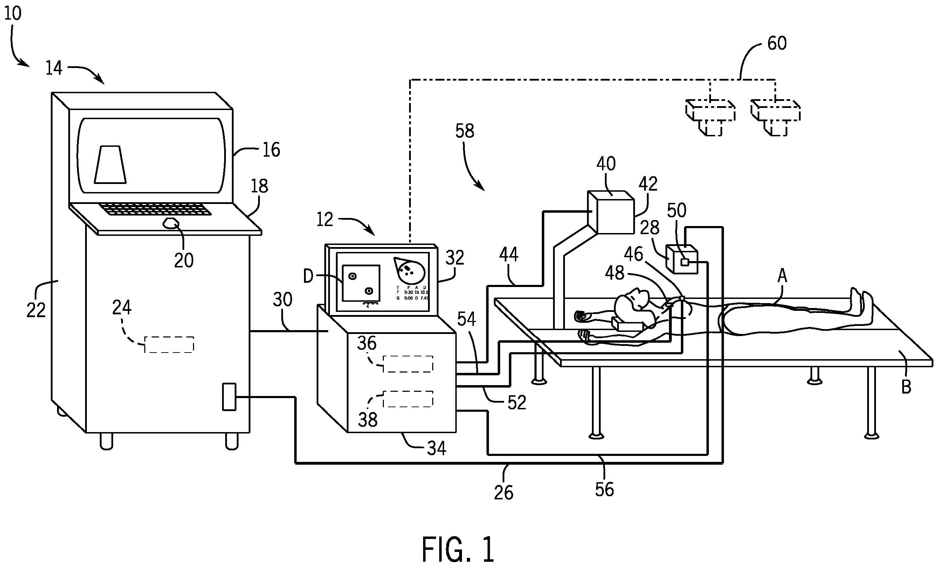

Turning to FIG. 1, a schematic illustration of an ultrasound system 10 incorporating a 3D mapping display system (TDMD) 12 is shown. Ultrasound system 10 includes an ultrasound machine 14 having a display 16, interface with keyboard 18 and pointer 20, chassis 22 containing operating hardware, which is referred to hereafter as a processor 24, probe connecting cord 26, and a handheld image data acquisition device or ultrasound probe or transducer 28 TDMD 12 is coupled to ultrasound system 10 by way of a video output cord 30. TDMD 12 may be deployed as an add-on to any existing ultrasound machine 14, and can outfit DICOM compatible and non-DICOM machines as well.

TDMD 12 includes a display 32, chassis 34 containing hardware, which is referred to hereafter as a processor 36, having programmed thereon software for positional tracking of anatomical references and image coregistration, a storage device 38, 3D magnetic tracking member 40 with the transmitter 42 connected to TDMD 12 by 3D magnetic tracking member cord 44. While both ultrasound machine 14 and TDMD 12 are illustrated as having individual displays 16, 32, it is contemplated that the visual outputs of ultrasound machine 14 and TDMD 12 may be combined in a single display in an alternative embodiment.

According to various embodiments, TDMD chassis 34 is a computer containing a processor 36 that is capable of running instructions compiled in C # and C++ languages. Alternatively, embodiments of the invention can be implemented with any suitable computer language, computer platform and operating system. Processor 36 is programmed with software that is used to process the data received by the processor 36 from the sensor assemblies 46, 48, 50 and data received from the ultrasound machine 14 and carry out the real time anatomical reference point tracking techniques described below that enable a user to accurately review, evaluate, and compare examination results by having anatomical reference(s) guides to isolate target sites. Processor 36 may also be programmed with image reconstruction software that permits TDMD 12 to receive data directly from the ultrasound transducer 28 and reconstruct 2D or 3D ultrasound images therefrom. The images are then provided to processor 36 of TDMD 12. In embodiments where ultrasound machine 14 generates analog images, an optional analog to digital video output module 16 (shown in phantom) is provided within processor 36 to digitize images received from ultrasound machine 14. One skilled in the art will recognize that video output module 16 may be omitted in embodiments incorporating an ultrasound machine 14 capable of providing digital images to TDMD 12. Processor 36 receives the digital ultrasound images, associates the associated positional information from positional sensor assemblies 46, 48, 50 with the image frames and/or a body diagram, and outputs the information to display 32 and/or to a storage device 38 for review and processing at a later time. Display 32 is then enabled to show images D captured by ultrasound device 14 and associated positional data as collected from sensor assemblies 46, 48, and 50. As used herein an "image frame", "ultrasound frame", or "ultrasound image frame" is synonymous with a 2D ultrasound image.

A first anatomical reference sensor assembly or positional sensor assembly 46 is connected to TDMD 12 by a cord 52 and is used to monitor the position of a first anatomical reference (AR) point on the patient's body A, such as the nipple C. Optionally, a second anatomical reference sensor assembly or positional sensor assembly 48 is attached to track the patient's body position in reference to the examination table B and is connected to TDMD 12 by a cord 54. In the exemplary embodiments described below, sensor assembly 48 is attached to a chest wall structure, such as, for example, the sternum. However, sensor assembly 46 and sensor assembly 48 may be positioned at alternative locations on the skin surface of the patient or within the patient during examination. As one non-limiting example, sensor assembly 46 may be positioned surrounding a mole or other mark at or just below the skin surface, allowing the sensor assembly 46 to be positioned at a reproducible location during repeat imaging sessions. Likewise, sensor assembly 48 may be positioned over a prominent spinous process of a cervical or thoracic vertebra, tip of acromio-clavicular joint, or other prominent and easily locatable anatomical feature. Another sensor assembly 50 is connected to ultrasound probe 28 and to TDMD 12 by a cord 56.

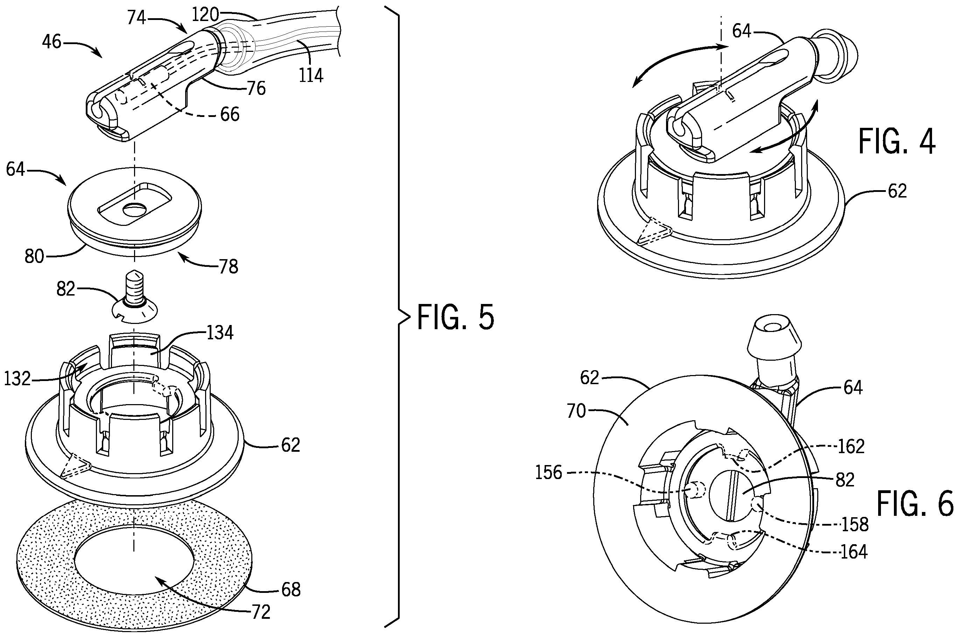

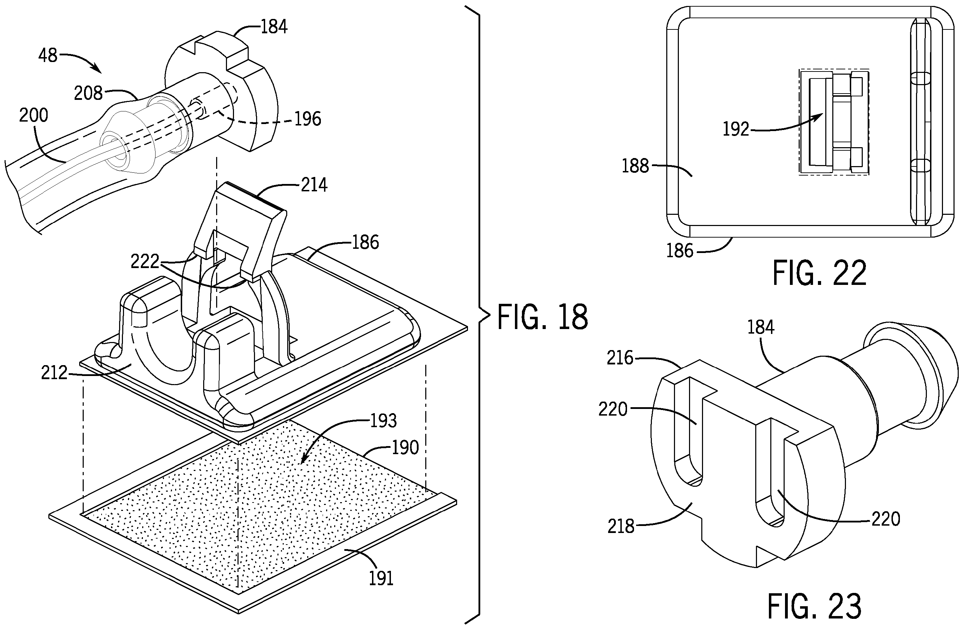

In the embodiments described herein, TDMD 12 integrates a 3D position registration system that is based on magnetic tracking technology. In such embodiments, TDMD 12, transmitter 42, and sensor assemblies 46, 48, and 50 operate together as a magnetic tracking system 58 and sensor assemblies 46, 48, and 50 include magnetic sensors such as, for example, magnetic sensors manufactured by Ascension Technology, Burlington, VT, which are capable of being tracked in three dimensions. However, any other suitable technology, such as optical or ultrasound, may be employed. With respect to sensor assemblies 46, 48 in particular, magnetic sensors 66, 196 are illustrated in FIGS. 5 and 18, respectively. Magnetic sensors 66, 196, which include three or more sensor coils (not shown), are used in combination with transmitter 42 to track the 3D position of the sensor assemblies 46, 48. Specifically, transmitter 42 generates controlled, varying magnetic fields, which induce voltages within the sensor coils of magnetic sensors 66, 196. The induced voltages are used by TDMD 12 to track the 3D position of the sensor assembly 46. Sensor assembly 50 operates in conjunction with transmitter 42 in a similar manner.

In an alternative embodiment, one or more of sensor assemblies 46, 48, and 50 are of a wireless variety, thus sensor cords, 52, 54, and/or 56 may be omitted. Also a combination of wired and wireless position sensors can be used to provide the position tracking module with positional information from tracked landmarks or anatomical reference (AR) points on the patient's body A and the ultrasound probe 28. In yet other embodiments, any of sensor assemblies 46, 48, and 50 may be replaced by markers capable of being tracked using an optional overhead infrared or optical AR tracking system 60 (shown in phantom), which incorporates one or more infrared or optical cameras. In such an embodiment, sensor cords 52, 54 would be omitted. When used, AR tracking system 60 may comprise at least one infrared camera, such as, for example, those commercially available (Natural Point Inc., Corvallis, OR), with the dedicated hardware and software receiving reflected infrared light from the reflectors or emitted infrared light from small infrared light sources applied over the anatomical references. The infrared cameras can be replaced with optical cameras and the infrared reflectors or emitters with optical markers or light emitters. Additionally, other tracking modalities like ultrasound, optical, inertial etc. can be used for the ultrasound probe and optical/pattern recognition, magnetic, etc. for the anatomical reference point real time tracking. It should also be noted that tracking modalities can be used in combination with one another, for non-limiting example, ultrasound tracking with optical tracking. It is also notable that the described TDMD system and method can optionally be used with the anatomical reference tracking feature disabled.

Referring again to FIG. 1, transmitter 42 and sensor assemblies 46, 48, 50 operate together to dynamically track the position of the ultrasound probe 28 and one or more AR points on or within the patient's body A. Transmitter 42 generates a magnetic field wherein sensor assemblies 46, 48, 50 operate and generate 3D positional outputs. All or any of sensor assemblies 46, 48, 50 provide positional information to the 3D position module within processor 36. The positional data received by TDMD 12 from sensor assemblies 46, 48, 50 is processed by processor 36 and used to co-register the ultrasound real time images acquired by ultrasound machine 14 with a body diagram or other secondary sets of acquired ultrasound images, to provide real time position and orientation information about the ultrasound probe 28, image frames, and the examined region of the patient's body A. Additional sensor assemblies or markers (not shown) may be included within TDMD 12 to track additional AR points on or within the patient's body A. For example, an additional sensor assembly constructed in a similar manner as sensor assembly 48 may be positioned at the skin surface on the back over a prominent spinous process of a cervical or thoracic vertebra. Positional data acquired using this additional sensor assembly may be used in connection with positional data acquired from sensor assembly 48 to recalculate the reference plane of the body, such as the sagittal plane, during the examination. According to various embodiments, TDMD 12 may be configured to continuously track one or several anatomical reference markers or sensors, which can increase the overall accuracy of the system. If multiple anatomical reference markers or sensors are used, TDMD 12 may track some or all of the markers or sensor assemblies continuously or intermittently.

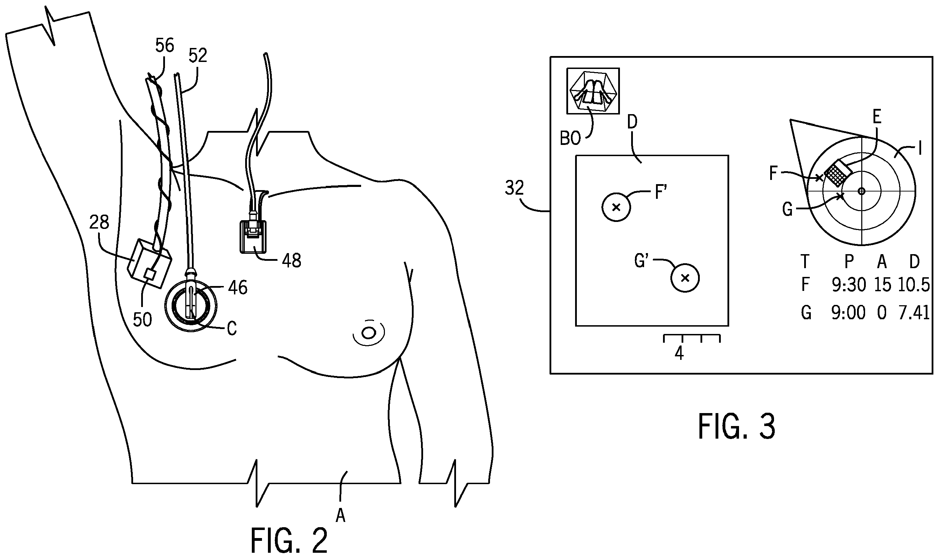

To ensure reproducible and accurate mapping of the ultrasound images, sensor assemblies 46, 48, 50 are attached at well-defined and reproducible sites, outside or inside the body A and on the ultrasound probe 28, respectively, during repeated ultrasound examinations. FIG. 2 is a schematic representation of a portion of the patient A, to illustrate exemplary positions of sensor assemblies 46, 48, and 50 during a breast ultrasound examination. As shown, sensor assembly 50 is coupled to ultrasound probe 28 and sensor assembly 46 is centered on the nipple C. In alternative embodiments, sensor assembly 46 may be positioned to the side of nipple C or positioned at alternative locations on the patient body A. Sensor assembly 46 continuously tracks the anatomical reference position, the nipple C in this case, to compensate for motion registration errors during the ultrasound examination. Likewise, sensor assembly 48 is positioned to track an alternative anatomical reference point on the patient's body A such as, for example, the sternum.

FIG. 3 illustrates TDMD display 32 having displayed thereon image D from the ultrasound machine 14 and the body part diagram I corresponding to FIG. 2, with the position and orientation of ultrasound probe 28 at the time of image capture D represented with icon E. The location of two different targets F and G are depicted in body part diagram I. The corresponding position of these targets are illustrated as F' and G' in image capture D. Additionally, each target F and G is displayed with the associated position (clock face position with hourly representation or degrees to longitudinal axis and anatomical reference as center) and distance (cm) from the selected anatomical reference. Positional coordinates are displayed under body part diagram I in FIG. 3. While TDMD 12 may display any number of coordinates, the non-limiting example in FIG. 3 illustrates the position of targets F and G in reference to the nipple C in hourly format (here, 9:30 for F and 9:00 for G), position from nipple C in degrees (here, 15° for F and 0° for G), and distance from nipple C in centimeters (cm) (here, 10.5cm for F and 7.41cm for G). When anatomical reference sensor assemblies 46 and 48 are used to dynamically track the position of the nipple C and patient's body A, the clock face position can be calculated in reference to the real time patient's body orientation planes, which would increase the accuracy and reproducibility of measured targets positional coordinates.

While represented as such in FIG. 3, the body diagram I is not limited to the two-dimensional (2D) "bird's eye view" type like the "clock" representation for the breast, but more complex and realistic 3D representations of the body or body regions, including images obtained with other modalities like MRI, mammograms, gamma cameras or positron emission tomography and using contour rendering algorithms, can be used. The calculated and recorded positional data can be displayed in these representations. Additionally, the position and orientation of ultrasound probe 28, can be depicted in a realistic appearance in space so it can be easily reproduced at subsequent examinations.

The position of a small tumor or other target in the breast, or other body part, depends on the patient's body position due to the gravity effect and the position and orientation of the ultrasound probe 28, which can displace the tissue under the probe 28 when pressure is applied by the operator on the ultrasound probe 28. To obtain accurate reproducible positional coordinates of a target or lesion from one examination to a subsequent examination, TDMD 12 measures the position and orientation of the ultrasound probe 28, monitors the patient's body position and orientation via sensor assembly 48 and displays it as icon BO (FIG. 3), and monitors for movement of deformable tissue via sensor assembly 46 in real time during an examination. In the case of a breast ultrasound exam, to obtain reproducible mapping results, sensor assembly 46 is attached to the nipple C and used to monitor the position of the same point at the nipple C, for example, the nipple center at the skin surface level, during repeated ultrasound exams. Because sensor assembly 46 is positioned on deformable tissue, sensor assembly 46 is designed such that it minimizes deformation of the skin surface and underlying tissue during examination while still providing the ability to account for any such deformation that occurs during the examination as a result of movement of the sensor assembly 46 itself or compression from the ultrasound probe 28.

Referring now to FIG. 4, a perspective view of magnetic sensor assembly 46 is illustrated according to one embodiment of the invention. Sensor assembly 46 includes a mounting base 62 and a sensor housing 64, the structural construction of which are described in detail with respect to FIGS. 4-15. To allow for a complete evaluation of the periareolar and retroareolar regions, mounting base 62 can be constructed from an ultrasound transparent material like TPX ® Polymethylpentene, or alternatively a non ultrasound transparent material, for example a thermoplastic such as Nylon 6/6 can be used. Sensor housing 64 is constructed of a thermoplastic polymer resin such as, for example, Delrin® acetal. Sensor housing 64 may be constructed of an ultrasound transparent material or a non-ultrasound transparent material in alternative embodiments. A magnetic sensor 66 is positioned within the sensor housing 64. In one embodiment, mounting base 62 is covered or embedded in an ultrasound transmitting soft pad (not shown) to aid in acoustic coupling and improved image quality of the retroareolar region.

In one embodiment an adhesive disk 68 is coupled to the bottom surface 70 of mounting base 62. Adhesive disk 68 has adhesive properties on both sides thereof to secure sensor assembly 46 mounting base 62 and to the skin surface of a patient. Optionally, adhesive disk 68 is provided with one or more removable release liners (not shown) to facilitate application to the skin surface of the patient. Adhesive disk 68 has an opening 72 formed in a center thereof, which permits adhesive disk 68 to be positioned surrounding the nipple C of the patient. Adhesive disk 68 can be made from an ultrasound transparent material, such as, for example, Tegaderm™, which prevents interference with ultrasound image acquisition, or can be made of any other double sided adhesive tape. In alternative embodiments, magnetic sensor assembly 46 may be secured to the skin surface using a medical grade adhesive, an alternative adhesive material, or an adhesive tape that covers the mounting base 62 and adjacent skin.

Referring now to FIGS. 4 -15 together as appropriate, the structural configuration of sensor assembly 46 will be described. Sensor housing 64 includes a top portion 74 comprising a protrusion 76 and a bottom portion 78 comprising a disk 80. In the illustrated embodiment, protrusion 76 and disk 80 are manufactured as separate components and coupled together with a fastener 82, which extends through an opening 84 formed through the disk 80 and is received within an opening 86 formed through the bottom surface 88 of the protrusion 76. Protrusion 76 seats within a recess 90 formed in a top surface 92 of disk 80. Alternatively, protrusion 76 and disk 80 may be permanently coupled to one another such as by an adhesive or a friction weld. In yet another alternative embodiment, protrusion 76 and disk 80 are manufactured as a single component.

Protrusion 76 includes a main body 94 having a cavity 96 formed therein that is sized to receive magnetic sensor 66. In the illustrated embodiment, magnetic sensor 66 is inserted into cavity 96 through a slot 98 formed in a top surface 100 of the main body 94. As shown, the width 102 of slot 98 is less than the width 104 of magnetic sensor 66, creating a snap fit connection that retains magnetic sensor 66 within sensor housing 64. An optional alignment marker 106 (shown in phantom) is formed on the top surface 100 of main body 94 perpendicular to the slot 98, which may be used to align magnetic sensor 66 within sensor housing 64 and facilitate the calibration of magnetic sensor 66 relative to sensor assembly 46. In an alternative embodiment, magnetic sensor 66 is embedded within main body 94 during the fabrication of sensor housing 64. In such an embodiment, slot 98 and alignment marker 106 may be omitted.

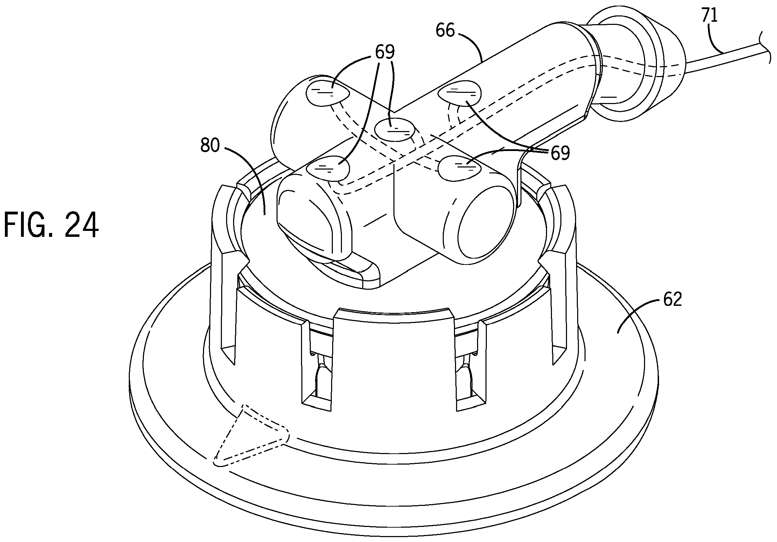

In yet another embodiment sensor housing 64 and magnetic sensor 66 may be replaced by a wired or wireless LED or infrared marker that is received within mounting base 62 and is configured to track the location of an anatomical reference point such as nipple C. Such an embodiment may include a sensor or marker housing 67 such as that shown in FIG. 24, which includes one or more LEDs 69 or optical devices powered through a wired connection 71 that extends through protrusion 66. In the illustrated embodiment, LEDs 69 are arranged in a cross pattern array that facilitates positional registration by optical tracking system 60 (FIG. 1). Similar to the magnetic sensor embodiment shown in FIG. 4, housing 67 includes a base portion or disk 80 configured to rotate within mounting base 62. In alternative embodiments, LEDs 69 may be replaced with one or more infrared markers. A rechargeable power source may be provided to power LEDs 69 or infrared marker(s) in lieu of wired connection 71. In wireless embodiments, the downward sloping end of protrusion 66 would be omitted.

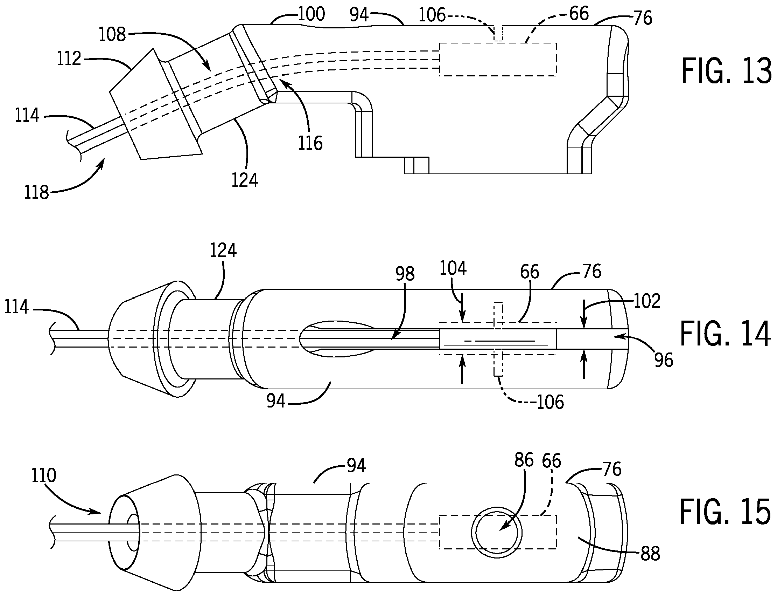

Sensor housing 64 is formed having a passage 108 that extends between cavity 96 and an opening 110 formed through an end portion 112 of the sensor housing 64. Multi-strand wire 114, which is coupled to the magnetic sensor 66, extends through the passage 108 and out through the opening 110. During the assembly process, the end of multi-strand wire 114 opposite magnetic sensor 66 is inserted into cavity 96 and threaded through opening 110. Magnetic sensor 66 is then aligned with cavity 96 and snap fit therein. As shown in FIGS. 13-15, once assembled a first portion 116 of multi-strand wire 114 is positioned within the passage 108 and a second portion 118 of multi-strand wire 114 extends outside of sensor housing 64 to couple with TDMD 12 (FIG. 1). The second portion 118 of the multi-strand wire 114 is encased in a tube 120. In one embodiment, tube 120 is a biocompatible polymer material such as, for example, Tygon®. Together tube 120 and multi-strand wire 114 form cord 54.

A first end 122 of the tube 120 fits over the end 112 of protrusion 76. As shown, end 112 includes a recessed channel 124 positioned adjacent an end portion 112 having a frustoconical geometry which function to retain tube 120 on sensor housing 64. The end 112 of protrusion 76 is oriented at a downward facing angle relative to main body 94. The downward facing orientation of end 112 minimizes deformation of the surface skin of the areola surrounding nipple C resulting from the combined weight of multi-strand wire 114 and tubing 120.

For practical purposes, it would be difficult to locate the magnetic sensor 66 within sensor housing 64 such that it would be positioned exactly at the center of the nipple C every time a patient is scanned. To address this limitation, magnetic sensor 66 is calibrated relative to mounting base 62 to track the center point of nipple C in the 3D reference frame. Such calibration can be performed using any known method to perform a point or stylus 3D calibration in a reference system, such as, for example, the calibration techniques described in U.S. Patent 9,119,585.

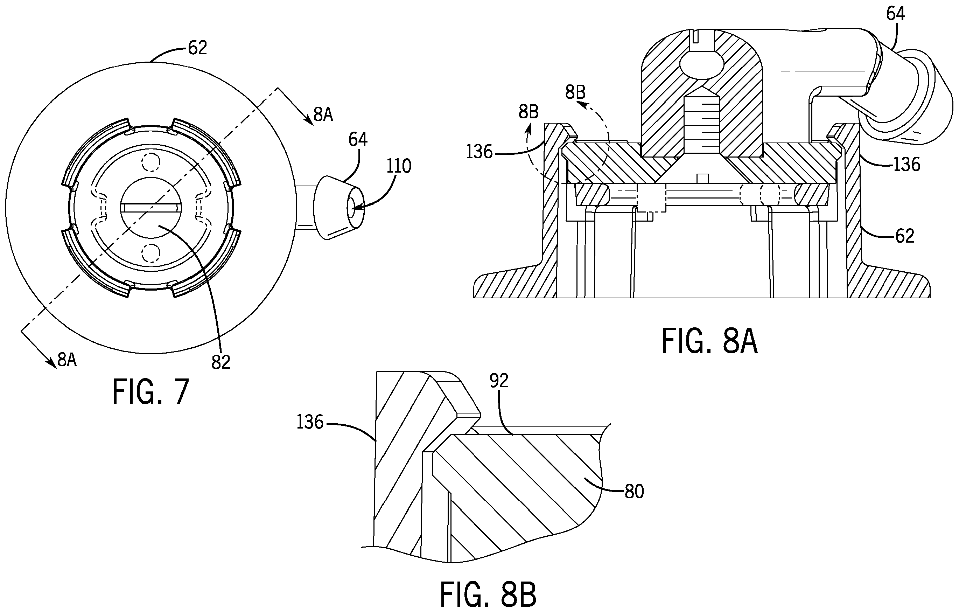

The outer perimeter 130 of the disk portion 80 of sensor housing 64 is sized to be received within a track 132 formed on an interior surface 134 of mounting base 62. Sensor housing 64 and mounting base 62 assemble together via a snap fit connection between at least one flexible tab 136 and disk 80. Thus, magnetic sensor assembly 48 is assembled by pressing sensor housing 64 downward into mounting base 62. Flexible tabs 136 extend upward from a collar 138 that is coupled to mounting flange 140, which forms the bottom surface 70 of mounting base 62. In the illustrated embodiment, mounting base 62 includes four flexible tabs 136 equally spaced around collar 138. However, it is contemplated that more or less tabs may be provided and the orientation thereof reconfigured based on design considerations. As shown in FIGS. 8A and 8B in particular, flexible tabs 136 are configured to engage a top surface 92 of disk 80. Sensor housing 64 is removed from mounting base 62 by pulling sensor housing 64 upwards and away from mounting base 62.

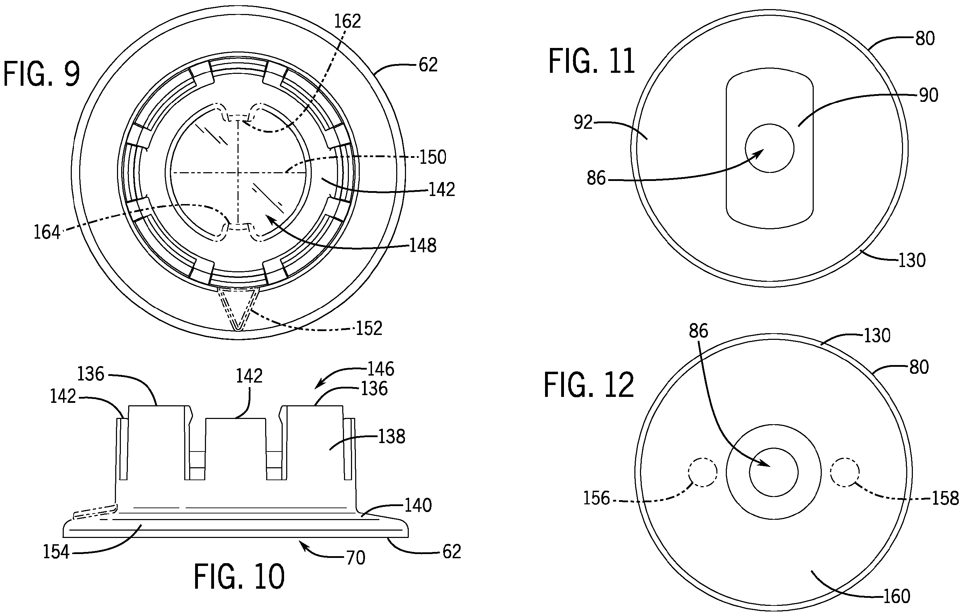

Support members 142 extend upward from collar 138 and are positioned between adjacent flexible tabs 136 as shown. A ledge 144 is coupled to the support members 142 at a location between a top surface 146 and a bottom surface 70. Ledge 144 provides a mounting platform for the disk 80 of sensor housing 64. When assembled, disk 80 is seated on the ledge 144 and sensor housing 64 rotates within track 132 in a plane parallel with disk 80. As shown in FIG. 9, an opening 148 is formed through the center of ledge 144, which facilitates alignment of the mounting base 62 relative the nipple C of the patient.

In one embodiment, mounting base 62 includes a transparent film printed with a cross-hair pattern 150 (shown in phantom) to further aid in centering mounting base 62 on the nipple C. Alternatively, a pattern of alignment marks (not shown) may be provided on ledge 144 or elsewhere on mounting base 62. Inclusion of a cross-hair or other alignment pattern may be of particular benefit in embodiments where sensor assembly 46 is centered over skin surface features, such as moles, during repeat examinations. Mounting base 62 may also include an optional alignment marker 152 (shown in phantom) provided on the top surface 154 of mounting flange 140, to indicate a direction in which mounting base 62 is to be positioned on patient - for example, to direct the clinician to position mounting base 62 such that alignment marker 152 is pointing toward the head of the patient.

The ability of sensor housing 64 to rotate relative to mounting base 62 allows unobstructed scanning of the breast tissue by permitting free movement of the multi-strand wire 114 and associated tube 120 during examination. In one embodiment, the angular rotation of sensor housing 64 relative to mounting base 62 is limited by engagement between a pair of studs 156, 158 that extend outward from the bottom surface 160 of disk 80 and a pair of projections or stops 162, 164 that extend outward from ledge 144. In an alternative embodiment mounting base 62 may be provided with a single stud. Studs 156, 158 are positioned relative to stops 162, 164 such that stud 156 impinges upon stop 144 and stud 158 impinges upon stop 164 when sensor housing 64 rotates in the clockwise direction. Likewise, studs 156, 158 impinge upon respective stops 164, 162 when sensor housing 64 rotates in the counterclockwise position. In the illustrated embodiment, studs 156, 158 and stops 164, 162 are positioned to limit rotation of sensor housing 64 relative to mounting base 62 to a total rotation of approximately 120 degrees. However, it is contemplated that the range of rotation may be varied in alternative embodiments. Providing this restricted range of rotation beneficially aids in preventing tubing 128 and multi-strand wire 114 from tangling and maintaining the accuracy in the calibration of magnetic sensor 66.

In an alternative embodiment sensor housing 64 and mounting base 62 may be reconfigured having a ball and socket engagement, permitting sensor housing 64 to rotate in three dimensions relative to mounting base 62 and allow multi-strand wire 114 to freely move in any direction during examination and minimize deformation of the skin surface resulting from the weight of the wire 114. In such an embodiment, disk 80 is replaced by a spherical or partially spherical structure that is received within a mating receptacle formed within mounting base 62.

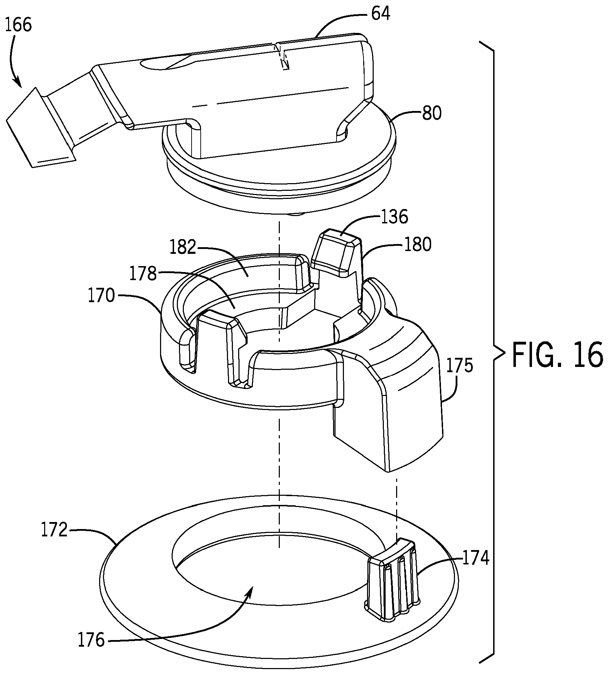

FIG. 16 illustrates a magnetic sensor assembly 166 for use in tracking an anatomical reference on the patient, such as the nipple C or reproducible skin surface feature, according to another embodiment of the invention. Magnetic sensor assembly 166 includes a sensor housing 64, similar to that of FIG. 4. It is contemplated that a magnetic sensor, multi-strand wire, and tubing would be incorporated with sensor housing 64 in a similar manner as described with respect to FIG. 4, but have been omitted from the illustration for purposes of clarity. Magnetic sensor assembly 166 also includes a mounting base 168 that receives sensor housing 64. Mounting base 168 includes a collar portion 170 that is offset from a mounting flange 172 by engagement between a post 174 positioned on mounting flange 172 and a receiving arm 175 coupled to collar portion 170. Together, an opening 176 provided in the center of mounting flange 172 and the spacing between mounting flange 172 and collar portion 170 permit mounting base 168 to be positioned atop and surrounding the nipple C of a patient during examination. An adhesive disk (not shown), similar to adhesive disk 68 of FIG. 4, may be used to couple mounting flange 172 to the skin surface of a patient.

Flexible tabs 136 releasably engage disk 80 of sensor housing 64, which seats on a ledge 178 formed within mounting base 168 and rotates within a track 180 formed on an inner surface 182 of the collar portion 170 of mounting base 168. In one embodiment, sensor housing 64 may be rotated within a full 360 range of rotation within mounting base 168. Alternatively, ledge 178 may include one or more stops similar to stops 162, 164 (FIG. 6) that engage one or more studs (not shown) positioned on the bottom surface of disk 80, to limit rotation of sensor housing 64 relative to mounting base 168.

During an ultrasound examination, the patient's body position and orientation to the examination table B or other fixed reference used to position the patient, a chair or a wall for example, can change, which can have an effect on the position of the body internal structures when referenced to other body landmarks in a spatial frame and therefore have an effect on the measurement and description of a lesion's position. During the real time ultrasound exam image acquisition and capture, each internal ultrasound target position relative to the body references depends, among other factors, on the patient's position relative to the direction of the gravity force or the earth's magnetic field. Therefore the positional relation between the patient's body position and the fixed reference can be associated with the ultrasound images or other images of the body to aid repositioning the patient at subsequent imaging and match the gravity force effect between temporally distinct image sets. The gravity force effect is larger on deformable structures, like the breast. For example, during a breast ultrasound exam, the position of a small target in the breast relative to the nipple or other anatomical reference can change between the supine and half decubitus patient positions on the examination table. Unlike the approaches of the prior art, at the follow up exams or during the same patient exam, the patient whole body position can be adjusted to match the body position relative to the examination table or other known fixed reference object recorded with the previously obtained ultrasound images and match the position of internal targets referenced to body landmarks in previous images, therefore help finding a target with the previously recorded coordinates relative to selected body landmarks.

To monitor the patient's body axes and planes in reference to an exam table, positional data acquired from one or more sensor assemblies can be used to calculate the patient's body position related to the examination table B. However, to assure the body position to the examination table B reproducibility at subsequent exams, the magnetic sensor tracking the body position would need to be reattached to the body in the same position referenced to the body, as it was at the first examination. This is a challenging task since small angular differences in the position of magnetic sensor on the body can generate significant changes in patient's planes and axes orientation and generate registration errors. For example, sensor assembly 46 attached to the nipple C could not be used to monitor changes in the patient's body position due to the breast's deformable nature and sensor position changes due to the ultrasound probe induced deformation, unrelated to the body position.

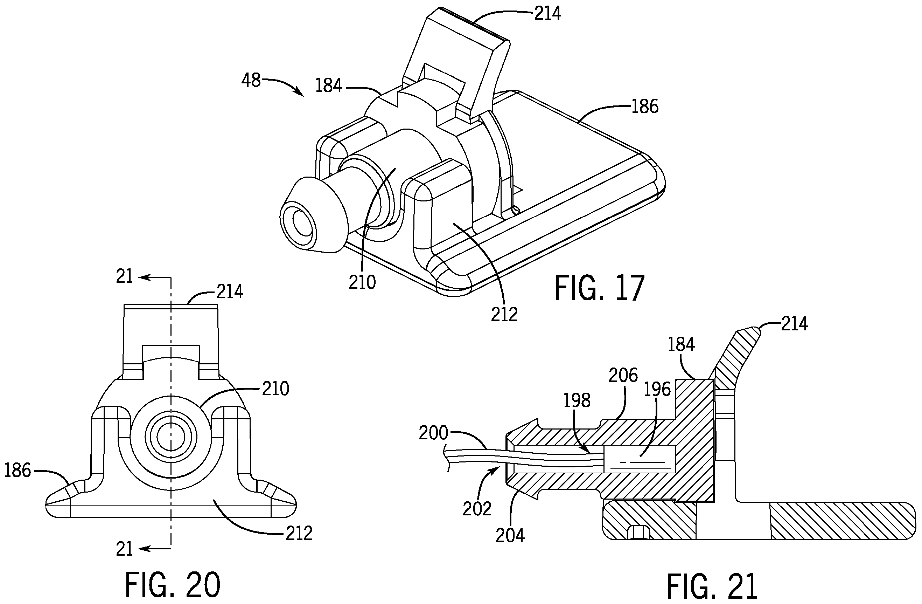

To address the above, additional magnetic sensor assembly 48, such as that shown in FIGS. 17 and 18 can be used in combination with magnetic sensor assembly 50 to track the real time position and orientation of the patient body during the examination. Magnetic sensor assembly 48 includes a sensor housing 184 and a mounting base 186, which is designed to be easily repositioned in same position and orientation at the patient's body during repeat examinations. In one embodiment, mounting base 186 has a substantially flat bottom surface 188 that may be attached directly to the skin surface of the patient using an adhesive pad 190, which may include removable release liners on its top and/or bottom surfaces. Mounting base 186 is coupled to the adhesive surface 193 of pad 190 after a first portion of the top release liner is removed, while a second portion 191 of the top release liner remains to facilitate handling. The bottom release liner (not shown) is removed prior to affixing adhesive pad 190 to the skin surface of the patient. Optionally, an opening 192 (shown in phantom in FIG. 22) is formed through the thickness of mounting base 186 to permit magnetic sensor assembly 48 to be oriented with respect to a particular marker or feature on the skin surface of the patient.

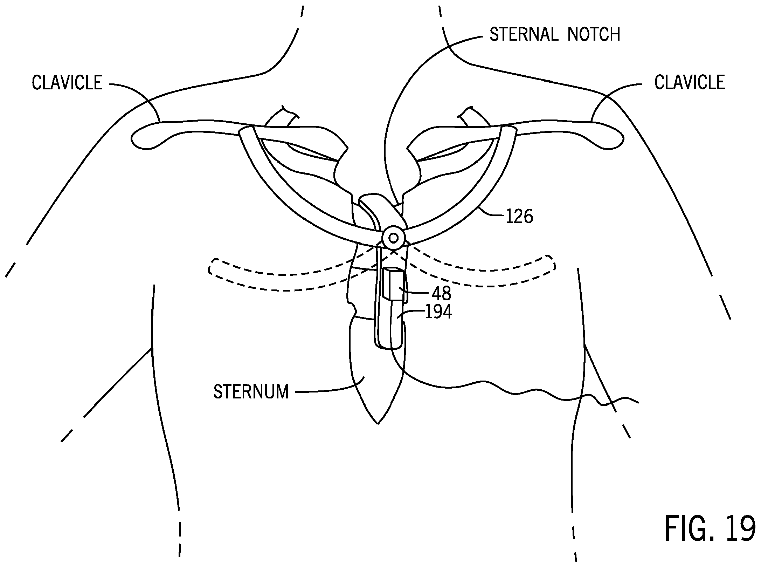

In an alternative embodiment, mounting base 186 is configured for attachment to an alignment member 194, shown in FIG. 19, which is designed as a long profile with a curved end which can be placed and aligned with the sternum long axis, with the curved end at the sternal notch or the xiphoid process of sternum. The position of alignment member 194 relative to the body can be stabilized with two extensions 120 attached to the clavicles. The extensions 126 ends may be taped at the clavicle and opposite ends firmly attached at alignment member 194 or attached to a pin which allows the extensions to rotate to fit the overlaying with the clavicle bone in a range of anatomical variants.

Referring now to FIGS. 17 and 18 in combination with FIGS. 20-23 as appropriate, additional features of magnetic sensor assembly 48 will be described. Similar to magnetic sensor assembly 46, a magnetic sensor 196 is positioned within a cavity 198 formed within sensor housing 184. A multi-strand wire 200 is coupled to magnetic sensor 196 and extends out through an opening 202 in the end 204 of the protrusion portion 206 of sensor housing 184 to provide positional data to TDMD 12 (FIG. 1). Biocompatible tubing 208 surrounds multi-strand wire 200 and is received on the end of sensor housing 184, which has a frustoconical geometry that aids in retaining tubing 208 thereon.

A central portion 210 of sensor housing 184 has a cylindrical geometry and is sized to seat within a U-shaped mounting platform 212 that extends upward from the mounting base 186. A flexible tab 214 also extends upward from the mounting base 186 and releasably engages a base portion 216 of sensor housing 184. As shown in FIGS. 18 and 23 in particular, the rear surface 218 of base portion 216 includes a pair of slots 220, which slide over corresponding projections 222 of flexible tab 214 to aid in alignment of sensor housing 184 relative to mounting base 186. Once sensor housing 184 is fully inserted within mounting base 186, projections 222 engage the top surface of base portion 216 and prevent sensor housing 184 from rotating within mounting base 186. After an examination is complete, sensor housing 184 may be separated by mounting base 186 by pulling flexible tab 214 away from base portion 216 of sensor housing 184.

Mounting base 186 and/or alignment member 194, when used, is calibrated to the attached magnetic sensor 196 using a known calibration technique, such as, for example, one of the calibration techniques described in U.S. Patent 9,119,585. As one example, calibration may be completed such that the long axis or the flat bottom plane of mounting base 186 is calibrated to magnetic sensor 196. The calibrated mounting base 186 can be used to define lines or planes which can be aligned with the body planes and axes, to facilitate repositioning mounting base 186 on the body during later examinations. Once attached to the sternum skin, the calibrated mounting base 186 and magnetic sensor 196 are used to track the patient's body long axes and planes.

In an alternative embodiment, the output from the sensor assembly 48 can be used to measure and set the body reference position and orientation with the patient's body positioned in the supine or other known reproducible body position on examination table B. After setting the patient's body reference planes in the spatial frame, the output from sensor assembly 48 can measure changes in the body position and orientation during the imaging session and the patient's whole body position relative to the examination table B or other fixed reference object can be recorded for each 2D ultrasound frame.

Alternatively, the patient's planes and axes can be measured using multiple anatomical references with the patient's body holding in one position and orientation on the examination table B. For example, longitudinal and transverse axes of the patient can be initially determined by recording the position of a chest wall structure such as the sternal notch via sensor assembly 48 and calculating the longitudinal and transverse axes of the patient in reference to the examination table or other fixed object, respectively. Sensor assembly 48 is aligned with the patient's body planes and axes and follows position and orientation changes of the body planes and axes during imaging. The output of sensor assembly 48 is registered with the positions of above-measured axes, planes or volume positions and the changes in the output of sensor assembly 48 is used to calculate positional changes in the patient's body axes or planes, which can be displayed in reference to another reference object, such as examination table B. Alternatively, the positional changes from the sensor assemblies 46, 48 attached to the patient A are applied to the patient body coordinates to display the whole body position change relative to the examination table B or other fixed reference. The positional change output from the sensor assemblies 46, 48 is applied to calculate the patient's planes position and orientation, and recorded with corresponding ultrasound images. The patient's real time body position during imaging (BO, FIG. 3) can be represented as the orthogonal imaginary axes and planes used to represent the whole patient body position, coronal plane, sagittal plane, axial plane or any other conventional representation.

Additionally, the rotation around the initial position of axes and planes can be graphically represented and recorded. The recorded body position from one or more previous images can be displayed in real time during a subsequent examination and used to reposition the body in the same position the previous images were obtained, to help produce the images in the same orientation and directions as those of previous images and help the relocation of previously detected targets and other associated findings with known positional coordinates relative to the anatomical references. Alternatively, if differences exist between the body position recorded with the previous images of same body region, the positional difference can be applied at the previous set of images to adjust the previous set of images positional data and display to guide the operator to match the real time images, with the previous set of images. This technique can be applied with a set of previously-acquired images and current images during scanning or to multiple sets of previously-acquired images to realign image sets recorded at different times.

These and additional methods for registering and recording the patient's body position are described in detail in U.S. Serial No. 13/719,200. With any method used for the patient's body position tracking, the recording of the patient's whole body position and orientation can be automated using TDMD 12 by tracking and recording the position coordinates of one or more anatomical reference sensors attached to the patient's body and compared with a reference body position coordinates. The real time or recorded images D can be displayed with the corresponding body position relative to the examination table B or other object in a body orientation diagram BO, together with the body diagram used to represent the relative position of the ultrasound probe 28, scanning plane, body diagram and any recorded targets, as shown in FIG. 3.

Beneficially, use of the positional sensor assemblies described herein eliminates the need for multiple fiducial markers and allows the registration of body volumes displayed within different image sets when using different anatomical landmarks to define the body planes and axes position and orientation in space.

One skilled in the art will appreciate that embodiments of the invention may be interfaced to and controlled by a computer readable storage medium having stored thereon a computer program. The computer readable storage medium includes a plurality of components such as one or more of electronic components, hardware components, and/or computer software components. These components may include one or more computer readable storage media that generally stores instructions such as software, firmware and/or assembly language for performing one or more portions of one or more implementations or embodiments of a sequence. These computer readable storage media are generally non-transitory and/or tangible. Examples of such a computer readable storage medium include a recordable data storage medium of a computer and/or storage device. The computer readable storage media may employ, for example, one or more of a magnetic, electrical, optical, biological, and/or atomic data storage medium. Further, such media may take the form of, for example, floppy disks, magnetic tapes, CD-ROMs, DVD-ROMs, hard disk drives, and/or electronic memory. Other forms of non-transitory and/or tangible computer readable storage media not list may be employed with embodiments of the invention.

A number of such components can be combined or divided in an implementation of a system. Further, such components may include a set and/or series of computer instructions written in or implemented with any of a number of programming languages, as will be appreciated by those skilled in the art. In addition, other forms of computer readable media such as a carrier wave may be employed to embody a computer data signal representing a sequence of instructions that when executed by one or more computers causes the one or more computers to perform one or more portions of one or more implementations or embodiments of a sequence.

Therefore, according to one embodiment of the invention, a positional sensor assembly includes a mounting base comprising at least one flexible tab, a mounting flange forming a bottom surface of the mounting base, and a ledge positioned between a top surface of the at least one flexible tab and the mounting flange. A sensor housing is positioned on the ledge and rotatably engaged with the mounting base. The sensor housing has a cavity sized to receive a positional sensor and the at least one flexible tab engages an upward-facing surface of the sensor housing.

According to another embodiment of the invention, a method of manufacturing a positional sensor assembly includes forming a mounting base having at least one flexible tab, a mounting flange, and a ledge positioned between the mounting flange and a top surface of the mounting base. The method also includes forming a sensor housing having a first portion sized to seat on the ledge of the mounting base and a second portion having a cavity formed therein to receive a positional sensor, the sensor housing rotatably engageable with the mounting base. The at least one flexible tab is sized to engage an upward-facing surface of the first portion of the sensor housing.

According to yet another embodiment of the invention, a 3D position tracking system includes a positional tracking system and a first sensor assembly having a mounting base comprising a bottom flange, at least one flexible tab, and a ledge positioned therebetween. A sensor housing is positioned on the ledge and rotatably engaged with the mounting base. A sensor is positioned within a cavity formed in the sensor housing and a sensor wires extending outward from an opening formed in the sensor housing, the sensor wire having a first end coupled to the magnetic sensor and a second end coupled to the positional tracking system. The positional tracking system is configured to track a real-time position of the sensor.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. An assembly (62, 64, 168) for placement of a positional sensor (66) for positional tracking of an anatomical reference point, the assembly (62, 64, 168) comprising a mounting base (62, 168) for placement over the anatomical reference point;
**characterized in that**:
the mounting base (62, 168) comprises:
at least one flexible tab (136);
a mounting flange (140, 172) forming a bottom surface of the mounting base (62, 168); and
a ledge (144, 178) positioned between a top surface of the at least one flexible tab (136) and the mounting flange (140, 172); and
a sensor housing (64) positioned on the ledge (144, 178) and rotatably engaged with the mounting base (62, 168), the sensor housing (64) having a cavity (96) sized to receive the positional sensor (66);
wherein the at least one flexible tab (136) engages an upward-facing surface (92) of the sensor housing (64).

2. The assembly of claim 1 wherein the sensor housing (64) comprises a protrusion (76) having the cavity (96) formed therein and a circular base (80); and
wherein the circular base (80) is received within a track (132) formed on an inner surface (134) of the mounting base (62, 168).

3. The assembly of claim 2 wherein the protrusion (76) has a downward sloping end (112) having a frustoconical geometry.

4. The assembly of claim 1 further comprising one of an optical marker and a magnetic sensor positioned within the cavity (96).

5. The assembly of claim 1 further comprising a stud (156, 158) extending outward from a bottom surface (160) of the sensor housing (64);
wherein the stud (156, 158) is positioned within an opening formed in the ledge (144, 178) and impinges upon a first stop (162, 164) extending from the ledge (144, 178) upon rotation of the sensor housing (64) in a clockwise direction and impinges upon a second stop (162, 164) extending from the ledge (144, 178) upon rotation of the sensor housing (64) in a counterclockwise direction.

6. The assembly of claim 4 further comprising a multi-strand sensor wire (114) coupled to the magnetic sensor, the multi-strand sensor wire (114) having a first portion (116) extending through a passage (108) formed between the cavity (96) and an opening (110) formed in an end (112) of the sensor housing (64) and a second portion (118) extending outward from the opening (110).

7. A method of manufacturing an assembly (62, 64, 168) for placement of a positional sensor (66) for positional tracking of an anatomical reference point, the method comprising:
forming a mounting base (62, 168) for placement over the anatomical reference point;
forming a sensor housing (64);
**characterized in that**:
the mounting base (62, 168) has at least one flexible tab (136), a mounting flange (140, 172), and a ledge (144, 178) positioned between the mounting flange (140, 172) and a top surface of the mounting base (62, 168); and
the sensor housing (64) has a first portion (80) sized to seat on the ledge (144, 178) of the mounting base (62, 168) and a second portion (76) having a cavity (96) formed therein to receive the positional sensor (66), the sensor housing (64) rotatably engageable with the mounting base (62, 168);
wherein the at least one flexible tab (136) is sized to engage an upward-facing surface (92) of the first portion of the sensor housing (64).

8. The method of claim 7 further comprising positioning the positional sensor (66) inside the cavity (96), the positional sensor (66) comprising one of a magnetic sensor and an optical marker.

9. The method of claim 8 further comprising feeding a multi-strand wire (114) coupled to the positional sensor (66) through a passage (108) formed between the cavity (96) and out an opening (110) formed in an end (112) of the sensor housing (64).

10. The method of claim 9 further comprising:
positioning a tube (120) to surround a portion of the multi-strand wire (114);
coupling an end (122) of the tube (120) to the end of the sensor housing (64); and
forming the sensor housing (64) having a downward sloping end (112).

11. The method of claim 7 further comprising forming a stud (156, 158) extending outward from a bottom surface (160) of the second portion of the sensor housing (64), the stud (156, 158) positioned to engage a pair of projections (162, 164) extending outward from the ledge (144, 178) and limit a range of rotation of the sensor housing (64) relative to the mounting base (62, 168).

12. A three-dimensional position tracking system comprising:
a positional tracking system (58); and
a first sensor assembly (46, 166) comprising:
the assembly (62, 64, 168) of any one of claims 1 to 6;
the positional sensor (66) positioned within the cavity (96) formed in the sensor housing (64); and
a sensor wire (114) extending outward from an opening (110) formed in the sensor housing, the sensor wire having a first end coupled to the positional sensor (66) and a second end coupled to the positional tracking system (58);
wherein the positional tracking system is configured to track a real-time position of the positional sensor (66).

13. The tracking system of claim 12 wherein a bottom portion (80) of mounting base (62, 168) of the first sensor assembly (46, 166) has a chamber formed therein sized to surround a nipple of a patient.

14. The tracking system of claim 12 wherein an end portion (112) of the sensor housing (64) is angled downward toward the mounting base (62, 168).

15. The tracking system of claim 12 further comprising a second sensor assembly (48) comprising:
a mounting base (186) comprising a flexible tab (214);
a sensor housing (184) releasably engaged by the mounting base (186) via the flexible tab (214);
a sensor (196) positioned within a cavity (198) formed in the sensor housing (184); and
a sensor wire (200) extending outward from an opening (202) formed in the sensor housing (184), the sensor wire (200) having a first end coupled to the sensor (196) and a second end coupled to the positional tracking system (58);
wherein the mounting base (186) of the second sensor assembly (48) preferably comprises a substantially planar bottom surface configured to mount on a sternum of a patient.

## Patentansprüche

1. Eine Anordnung (62, 64, 168) zur Platzierung eines Positionssensors (66) zur Positionsverfolgung bzw. zum Positionstracking eines anatomischen Referenzpunktes, wobei die Anordnung (62, 64, 168) eine Befestigungsbasis bzw. -platte (62, 168) zur Platzierung über dem anatomischen Referenzpunkt aufweist;
**dadurch gekennzeichnet, dass**:
die Befestigungsbasis (62, 168) Folgendes aufweist:
mindestens ein(e) flexible(s) Tab bzw. Lasche (136);
einen Befestigungsflansch (140, 172), der eine Bodenfläche der Befestigungsbasis (62, 168) bildet; und
eine Leiste (144, 178), die zwischen einer Oberseite der mindestens einen flexiblen Lasche (136) und dem Befestigungsflansch (140, 172) angeordnet ist; und
ein Sensorgehäuse (64), das auf der Leiste (144, 178) positioniert ist und das drehbar mit der Befestigungsbasis (62, 168) in Eingriff steht, wobei das Sensorgehäuse (64) einen Hohlraum (96) aufweist, der so bemessen ist, dass er den Positionssensor (66) aufnimmt;
wobei die mindestens eine flexible Lasche (136) mit einer nach oben weisenden Oberfläche (92) des Sensorgehäuses (64) in Eingriff steht.

2. Die Anordnung nach Anspruch 1, wobei das Sensorgehäuse (64) einen Vorsprung (76) mit dem darin ausgebildeten Hohlraum (96) und eine kreisförmige Platte bzw. Basis (80) aufweist; und
wobei die kreisförmige Basis (80) in einer auf einer Innenfläche (134) der Befestigungsbasis (62, 168) ausgebildeten Spur (132) aufgenommen ist.

3. Die Anordnung nach Anspruch 2, wobei der Vorsprung (76) ein nach unten geneigtes Ende (112) mit einer kegelstumpfförmigen Geometrie aufweist.

4. Die Anordnung nach Anspruch 1, die ferner eine optische Markierung bzw. einen optischen Marker oder einen magnetischen Sensor aufweist, der in dem Hohlraum (96) angeordnet ist.

5. Die Anordnung nach Anspruch 1, die ferner einen Zapfen (156, 158) aufweist, der sich von einer Bodenfläche (160) des Sensorgehäuses (64) nach außen erstreckt;
wobei der Zapfen (156, 158) in einer in der Leiste (144, 178) ausgebildeten Öffnung positioniert ist und auf einen ersten Anschlag (162, 164) auftrifft, der sich von der Leiste (144, 178) weg erstreckt, und zwar bei Drehung des Sensorgehäuses (64) im Uhrzeigersinn, und wobei der Zapfen auf einen zweiten Anschlag (162, 164) auftrifft, der sich von der Leiste (144, 178) weg erstreckt, und zwar bei Drehung des Sensorgehäuses (64) gegen den Uhrzeigersinn.

6. Die Anordnung nach Anspruch 4, die ferner einen mehrsträngigen Sensordraht bzw. ein mehrsträngiges Sensorkabel (114) aufweist, das an den magnetischen Sensor gekoppelt ist, wobei das mehrsträngige Sensorkabel (114) einen ersten Abschnitt (116) aufweist, der sich durch einen Durchgang (108) erstreckt, der zwischen dem Hohlraum (96) und einer in einem Ende (112) des Sensorgehäuses (64) ausgebildeten Öffnung (110) gebildet ist, und einen zweiten Abschnitt (118), der sich von der Öffnung (110) nach außen erstreckt.

7. Ein Verfahren zur Herstellung einer Anordnung (62, 64, 168) zur Platzierung eines Positionssensors (66) zur Positionsverfolgung bzw. zum Positionstracking eines anatomischen Referenzpunktes, wobei das Verfahren Folgendes aufweist:
Bilden einer Befestigungsplatte bzw. -basis (62, 168) zur Platzierung über dem anatomischen Referenzpunkt;
Bilden eines Sensorgehäuses (64);
**dadurch gekennzeichnet, dass**:
die Befestigungsbasis (62, 168) mindestens eine flexible Lasche bzw. ein flexibles Tab (136), einen Montageflansch (140, 172) und eine Leiste (144, 178) aufweist, die zwischen dem Montageflansch (140, 172) und einer oberen Fläche der Befestigungsbasis (62, 168) positioniert ist; und
das Sensorgehäuse (64) einen ersten Abschnitt (80) aufweist, der so bemessen ist, dass er auf der Leiste (144, 178) der Befestigungsbasis (62, 168) sitzt, und einen zweiten Abschnitt (76) mit einem darin ausgebildeten Hohlraum (96) zur Aufnahme des Positionssensors (66), wobei das Sensorgehäuse (64) drehbar mit der Befestigungsbasis (62, 168) in Eingriff bringbar ist;
wobei die mindestens eine flexible Lasche (136) so bemessen ist, dass sie mit einer nach oben weisenden Oberfläche (92) des ersten Abschnitts des Sensorgehäuses (64) in Eingriff kommt.

8. Das Verfahren nach Anspruch 7, das ferner Positionieren des Positionssensors (66) in dem Hohlraum (96) aufweist, wobei der Positionssensor (66) einen magnetischen Sensor oder eine optische Markierung bzw. einen optischen Marker aufweist.

9. Das Verfahren nach Anspruch 8, das ferner Zuführen eines mehrsträngigen Drahtes bzw. Kabels (114) gekoppelt an den Positionssensor (66) aufweist, und zwar durch einen Durchgang (108), der zwischen dem Hohlraum (96) und einer Öffnung (110), die in einem Ende (112) des Sensorgehäuses (64) ausgebildet ist.

10. Das Verfahren nach Anspruch 9, das ferner Folgendes aufweist:
Positionieren eines Rohrs (120), um einen Teil des mehrsträngigen Kabels (114) zu umgeben;
Koppeln eines Endes (122) des Rohrs (120) an das Ende des Sensorgehäuses (64); und
Bilden des Sensorgehäuses (64), das ein nach unten geneigtes Ende (112) aufweist.

11. Das Verfahren nach Anspruch 7, das ferner Bilden eines Zapfens (156, 158) aufweist, der sich von einer Bodenfläche (160) des zweiten Abschnitts des Sensorgehäuses (64) nach außen erstreckt, wobei der Zapfen (156, 158) so positioniert ist, dass er mit einem Paar von Vorsprüngen (162, 164) in Eingriff kommt, die sich von der Leiste (144, 178) nach außen erstrecken und die einen Drehbereich des Sensorgehäuses (64) relativ zu der Befestigungsbasis (62, 168) begrenzen.

12. Ein 3D-Positionsverfolgungs- bzw. -trackingsystem, das Folgendes aufweist:
ein Positionsverfolgungs- bzw. trackingsystem (58); und
eine erste Sensoranordnung (46, 166), die Folgendes aufweist:
die Anordnung (62, 64, 168) nach einem der Ansprüche 1 bis 6;
den Positionssensor (66), angeordnet in dem im Sensorgehäuse (64) ausgebildeten Hohlraum (96); und
einen Sensordraht bzw. ein Sensorkabel (114), das sich von einer in dem Sensorgehäuse ausgebildeten Öffnung (110) nach außen erstreckt, wobei das Sensorkabel ein erstes Ende aufweist, das mit dem Positionssensor (66) gekoppelt ist, und ein zweites Ende, das mit dem Positionsverfolgungssystem (58) gekoppelt ist;
wobei das Positionsverfolgungssystem eingerichtet ist, eine Echtzeit-Position des Positionssensor (66) zu verfolgen.

13. Das Positionsverfolgungsystem nach Anspruch 12, wobei ein unterer Abschnitt (80) der Befestigungsbasis (62, 168) der ersten Sensoranordnung (46, 166) eine darin ausgebildete Kammer aufweist, die so bemessen ist, dass sie einen Nippel eines Patienten umgibt.

14. Das Positionsverfolgungsystem nach Anspruch 12, wobei ein Endabschnitt (112) des Sensorgehäuses (64) nach unten in Richtung der Befestigungsbasis (62, 168) abgewinkelt ist.

15. Das Positionsverfolgungsystem nach Anspruch 12, das ferner eine zweite Sensoranordnung (48) aufweist, die Folgendes aufweist:
eine Befestigungsbasis bzw. -platte (186), die eine flexible Lasche bzw. ein flexibles Tab (214) aufweist;
ein Sensorgehäuse (184), das über die flexible Lasche (214) mit der Befestigungsbasis (186) lösbar in Eingriff steht;
einen Sensor (196), der in dem im Sensorgehäuse (184) gebildeten Hohlraum (198) positioniert ist; und
einen Sensordraht bzw. ein Sensorkabel (200), das sich von einer in dem Sensorgehäuse (184) ausgebildeten Öffnung (202) nach außen erstreckt, wobei das Sensorkabel (200) ein erstes Ende aufweist, das an den Sensor (196) gekoppelt ist, und ein zweites Ende, das an das Positionsverfolgungssystem (58) gekoppelt ist;
wobei die Befestigungsbasis (186) der zweiten Sensoranordnung (48) vorzugsweise eine im Wesentlichen ebene Bodenfläche aufweist, die eingerichtet ist, an einem Sternum eines Patienten befestigt zu werden.

## Revendications

1. Ensemble (62, 64, 168) destiné au positionnement d'un capteur de position (66) destiné au suivi de position d'un point de référence anatomique, l'ensemble (62, 64, 168) comprenant une base de montage (62, 168) destiné au positionnement au-dessus du point de référence anatomique,
**caractérisé en ce que** :
la base de montage (62, 168) comprend :
au moins une patte flexible (136) ;
une bride de montage (140, 172) formant une surface inférieure de la base de montage (62, 168) ; et
un rebord (144, 178) placé entre une surface supérieure de ladite au moins une patte flexible (136) et la bride de montage (140, 172) ; et
un boîtier de capteur (64) placé sur le rebord (144, 178) et engagé en rotation avec la base de montage (62, 168), le boîtier de capteur (64) ayant une cavité (96) dimensionnée pour recevoir le capteur de position (66) ;
dans lequel ladite au moins une patte flexible (136) s'engage avec une surface tournée vers le haut (92) du boîtier de capteur (64).

2. Ensemble selon la revendication 1, dans lequel le boîtier de capteur (64) comprend une saillie (76) ayant la cavité (96) formée en elle et une base circulaire (80) ; et
dans lequel la base circulaire (80) est reçue à l'intérieur d'une piste (132) formée sur une surface intérieure (134) de la base de montage (62, 168).

3. Ensemble selon la revendication 2, dans lequel la saillie (76) a une extrémité inclinée vers le bas (112) ayant une géométrie tronconique.

4. Ensemble selon la revendication 1, comprenant en outre un marqueur optique et un capteur magnétique placé à l'intérieur de la cavité (96).

5. Ensemble selon la revendication 1, comprenant en outre un goujon (156, 158) s'étendant vers l'extérieur à partir d'une surface inférieure (160) du boîtier de capteur (64) ;
dans lequel le goujon (156, 158) est placé à l'intérieur d'une ouverture formée dans le rebord (144, 178) et heurte une première butée (162, 164) s'étendant à partir du rebord (144, 178) pendant la rotation du boîtier de capteur (64) dans le sens des aiguilles d'une montre et heurte une deuxième butée (162, 164) s'étendant à partir du rebord (144, 178) pendant la rotation du boîtier de capteur (64) dans un sens antihoraire.

6. Ensemble selon la revendication 4, comprenant en outre un fil de capteur multibrins (114) couplé au capteur magnétique, le fil de capteur multibrins (114) ayant une première partie (116) s'étendant à travers un passage (108) formé entre la cavité (96) et une ouverture (110) formée dans une extrémité (112) du boîtier de capteur (64) et une deuxième partie (118) s'étendant vers l'extérieur à partir de l'ouverture (110).

7. Procédé de fabrication d'un ensemble (62, 64, 168) destiné au positionnement d'un capteur de position (66) destiné au suivi de position d'un point de référence anatomique, le procédé comprenant les étapes suivantes :
la formation d'une base de montage (62, 168) destiné au positionnement au-dessus du point de référence anatomique ;
la formation d'un boîtier de capteur (64) ;
**caractérisé en ce que** :
la base de montage (62, 168) a au moins une patte flexible (136), une bride de montage (140, 172), et un rebord (144, 178) placé entre la bride de montage (140, 172) et une surface supérieure de la base de montage (62, 168) ; et
le boîtier de capteur (64) a une première partie (80) dimensionnée pour être placée sur le rebord (144, 178) de la base de montage (62, 168) et une deuxième partie (76) ayant une cavité (96) formée dans celle-ci pour recevoir le capteur de position (66), le boîtier de capteur (64) pouvant s'engager en rotation avec la base de montage (62, 168),
dans lequel ladite au moins une patte flexible (136) est dimensionnée pour s'engager avec une surface tournée vers le haut (92) de la première partie du boîtier de capteur (64).

8. Procédé selon la revendication 7, comprenant en outre le positionnement du capteur de position (66) à l'intérieur de la cavité (96), le capteur de position (66) comprenant un élément parmi un capteur magnétique et un marqueur optique.

9. Procédé selon la revendication 8, comprenant en outre la fourniture d'un fil multibrins (114) couplé au capteur de position (66) à travers un passage (108) formé entre la cavité (96) et une ouverture (110) formée dans une extrémité (112) du boîtier de capteur (64).

10. Procédé selon la revendication 9, comprenant en outre les étapes suivantes :
le positionnement d'un tube (120) pour entourer une partie du fil multibrins (114) ;
le couplage d'une extrémité (122) du tube (120) à l'extrémité du boîtier de capteur (64) ; et
la formation du boîtier de capteur (64) ayant une extrémité inclinée vers le bas (112).

11. Procédé selon la revendication 7, comprenant en outre la formation d'un goujon (156, 158) s'étendant vers l'extérieur à partir d'une surface inférieure (160) de la deuxième partie du boîtier de capteur (64), le goujon (156, 158) étant positionné pour s'engager avec une paire de saillies (162, 164) s'étendant vers l'extérieur à partir du rebord (144, 178) et limiter une plage de rotation du boîtier de capteur (64) par rapport à la base de montage (62,168).

12. Système de suivi de position en trois dimensions comprenant :
un système de suivi de position (58) ; et
un premier ensemble capteur (46, 166) comprenant :
l'ensemble (62, 64, 168) selon l'une quelconque des revendications 1 à 6 ;
le capteur de position (66) positionné à l'intérieur de la cavité (96) formée dans le boîtier de capteur (64) ; et
un fil de capteur (114) s'étendant vers l'extérieur à partir d'une ouverture (110) formée dans le boîtier de capteur, le fil de capteur ayant une première extrémité couplée au capteur de position (66) et une deuxième extrémité couplée au système de suivi de position (58) ;
dans lequel le système de suivi de position est configuré pour suivre une position en temps réel du capteur de position (66).

13. Système de suivi selon la revendication 12, dans lequel une partie inférieure (80) de la base de montage (62, 168) du premier ensemble capteur (46, 166) a une chambre formée dans celle-ci dimensionnée pour entourer un mamelon d'un patient.

14. Système de suivi selon la revendication 12, dans lequel une partie d'extrémité (112) du boîtier de capteur (64) est inclinée vers le bas en direction de la base de montage (62, 168) .

15. Système de suivi selon la revendication 12, comprenant en outre un deuxième ensemble capteur (48) comprenant :
une base de montage (186) comprenant une patte flexible (214) ;
un boîtier de capteur (184) engagé de façon amovible par la base de montage (186) via la patte flexible (214) ;
un capteur (196) positionné à l'intérieur d'une cavité (198) formée dans le boîtier de capteur (184) ; et
un fil de capteur (200) s'étendant vers l'extérieur à partir d'une ouverture (202) formée dans le boîtier de capteur (184), le fil de capteur (200) ayant une première extrémité couplée au capteur (196) et une deuxième extrémité couplée au système de suivi de position (58) ;
dans lequel la base de montage (186) du deuxième ensemble capteur (48) comprend, de préférence, une surface inférieure sensiblement plane configurée pour être montée sur le sternum d'un patient.
